# EUROPEAN PATENT SPECIFICATION

(11) **EP 2 508 617 B1**
(45) Date of publication and mention of the grant of the patent: **24.06.2015**
(21) Application number: 10834668.5
(22) Date of filing: 03.12.2010
(51) Int. Cl.: C12Q 1/68, C12N 15/09

(54) **KIT AND METHOD FOR IDENTIFICATION OF CAUSATIVE BACTERIUM OF NAIL TINEA**
KIT UND VERFAHREN ZUR IDENTIFIKATION VON NAGELFLECHTE VERURSACHENDEN BAKTERIEN
TROUSSE ET PROCÉDÉ D'IDENTIFICATION DE LA BACTÉRIE CAUSALE DE LA TEIGNE DE L'ONGLE

(30) Priority: 04.12.2009 JP 2009276711
(43) Date of publication of application: 10.10.2012
(73) Proprietor: HISAMITSU PHARMACEUTICAL CO., INC., Saga 841-0017 (JP)
(72) Inventor: MAKIMURA, Koichi, Hachioji-shi Tokyo 192-0395 (JP); MIYAJIMA, Yoshiharu, Hachioji-shi Tokyo 192-0395 (JP); WATANABE, Shinichi, Tokyo 173-8606 (JP)
(74) Representative: Grünecker Patent- und Rechtsanwälte PartG mbB
(86) International application number: PCT/JP2010/071727
(87) International publication number: WO 2011/068218

(56) References cited:
- WO-A2-2006/133701
- JP-A- 2008 278 871
- US-A1- 2009 081 666
- A. M. C. BERGMANS ET AL: "Evaluation of a single-tube real-time PCR for detection and identification of 11 dermatophyte species in clinical material", CLINICAL MICROBIOLOGY AND INFECTION, vol. 16, no. 6, 23 September 2009 (2009-09-23), pages 704-710, XP055058702, ISSN: 1198-743X, DOI: 10.1111/j.1469-0691.2009.02991.x
- M. ARABATZIS ET AL: "Diagnosis of common dermatophyte infections by a novel multiplex real-time polymerase chain reaction detection/identification scheme", BRITISH JOURNAL OF DERMATOLOGY, vol. 157, no. 4, 1 October 2007 (2007-10-01), pages 681-689, XP055037619, ISSN: 0007-0963, DOI: 10.1111/j.1365-2133.2007.08100.x
- MOCHIZUKI TAKASHI ET AL: "Identification of Several Clinical Isolates of Dermatophytes Based on the Nucleotide Sequence of Internal Transcribed Spacer 1 (ITS 1) in Nuclear Ribosomal DNA", JOURNAL OF DERMATOLOGY, JAPANESE DERMATOLOGICAL ASSOCIATION, TOKYO, JP, vol. 26, no. 5, 1 August 1999 (1999-08-01) , pages 276-281, XP008156411, ISSN: 0385-2407
- MAKIMURA KOICHI: "Species Identification System for Dermatophytes Based on the DNA Sequences of Nuclear Ribosomal Internal Transcribed Spacer 1", JAPANESE JOURNAL OF MEDICAL MYCOLOGY / NIPPON ISHINKIN GAKKAI ZASSHI, NIHON ISHINKIN GAKKAI, JP, vol. 42, 30 April 2001 (2001-04-30), pages 61-67, XP008156399, ISSN: 0916-4804
- A. M. C. BERGMANS ET AL: "Validation of PCR-reverse line blot, a method for rapid detection and identification of nine dermatophyte species in nail, skin and hair samples", CLINICAL MICROBIOLOGY AND INFECTION, vol. 14, no. 8, 1 August 2008 (2008-08-01) , pages 778-788, XP055058704, ISSN: 1198-743X, DOI: 10.1111/j.1469-0691.2008.02036.x
- YOSHIHARU MIYAJIMA ET AL: "Rapid real-time diagnostic PCR for Trichophyton rubrum and Trichophyton mentagrophytes in patients with tinea unguium and tinea pedis using specific fluorescent probes", JOURNAL OF DERMATOLOGICAL SCIENCE, vol. 69, no. 3, 1 March 2013 (2013-03-01), pages 229-235, XP055058788, ISSN: 0923-1811, DOI: 10.1016/j.jdermsci.2012.11.589
- ARABATZIS M. ET AL.: 'Diagnosis of common dermatophyte infections by a novel multiplex real-time polymerase chain reaction detection/ identification scheme' BRITISH JOURNAL OF DERMATOLOGY vol. 157, 2007, pages 681 - 689, XP055037619
- MAKIMURA KOICHI: 'Species Identification System for Dermatophytes Based on the DNA Sequences of Nuclear Ribosomal Internal Transcribed Spacer 1.' JAPANESE JOURNAL OF MEDICAL MYCOLOGY vol. 42, 30 April 2001, pages 61 - 67, XP008156399
- YOSHIDA ERI. ET AL.: 'Rapid identification of Trichophyton tonsurans by specific PCR based on DNA sequences of nuclear ribosomal internal transcribed spacer (ITS) 1 region' JOURNAL OF DERMATOLOGICAL SCIENCE vol. 42, 2006, pages 225 - 230, XP027954762
- MOCHIZUKI TAKASHI. ET AL.: 'Identification of Several Clinical Isolates of Dermatophytes Based on the Nucleotide Sequence of Internal Transcribed Spacer 1 (ITS 1) in Nuclear Ribosomal DNA.' THE JOURNAL OF DERMATOLOGY vol. 26, 1999, pages 276 - 281, XP008156411

### [Sequence Listing]

### SEQUENCE LISTING

<110> Hisamitsu Pharmaceutical Co., Inc.
<120> Kit and Method for Identifying Causative Fungi of Tinea Unguium
<130> 1407-PCT
<150> JP 2009-276711
   <151> 2009-12-04
<160> 10
<170> PatentIn version 3.1
<210> 1
   <211> 25
   <212> DNA
   <213> Artificial
<220>
   <223> Forward Primer
<400> 1
   taacaaggtt tccgtaggtg aacct 25
<210> 2
   <211> 19
   <212> DNA
   <213> Artificial
<220>
   <223> Reverse Primer
<400> 2
   tcgctgcgtt cttcatcga 19
<210> 3
   <211> 23
   <212> DNA
   <213> Artificial
<220>
   <223> Forward Primer
<400> 3
   ssccccattc ttgtctacmt yac 23
<210> 4
   <211> 23
   <212> DNA
   <213> Artificial
<220>
   <223> Reverse Primer
<400> 4
   aacgctcaga ctgacagctc ttc 23
<210> 5
   <211> 19
   <212> DNA
   <213> Artificial
<220>
   <223> probe for Trichophyton mentagrophytes
<400> 5
   ctctctttag tggctaaac 19
<210> 6
   <211> 14
   <212> DNA
   <213> Artificial
<220>
   <223> probe for Trichophyton rubrum
<400> 6
   cgcgctcccc ctgc 14
<210> 7
   <211> 17
   <212> DNA
   <213> Artificial
<220>
   <223> universal probe for fungi
<400> 7
   ttyaacaayg gatctct 17
<210> 8
   <211> 18
   <212> DNA
   <213> Artificial
<220>
   <223> Forward Primer
<400> 8
   ggctgctcgc ggataccc 18
<210> 9
   <211> 19
   <212> DNA
   <213> Artificial
<220>
   <223> Reverse Primer
<400> 9
   tgagggaatg tgggaaccg 19
<210> 10
   <211> 24
   <212> DNA
   <213> Artificial
<220>
   <223> probe
<400> 10
   acctcgggtt tccgtcttgc tcgt 24

## Description

### Technical Field

The present invention relates to a kit and method for identification of causative fungi of tinea unguium (ringworm of the nail), using real-time PCR.

### Background Art

Tinea unguium is caused by fungal infection of the nails in more than 90% of cases, and when serious it leads to discoloration of the nails to white or yellowish-brown, thickening, and separation from the nail bed. A number of causative fungi of tinea unguium exist, most of which are of two species, *Trichophyton rubrum* and *Trichophyton mentagrophytes* (Non-patent document 1). Treatment of tinea unguium employs oral antifungal drugs such as terbinafine, itraconazole and griseofulvin, and topical antifungal drugs such as imidazole-based, allylamine-based, benzylamine-based and thiocarbamic acid-based drugs. However, tinea unguium is generally more difficult to cure than tinea pedis (ringworm of the foot) or tinea corporis (ringworm of the body), and it is a particular problem that the existing topical antifungal drugs have low efficacy. In order to improve the curative effect for tinea unguium, it is essential to rapidly identify the causative fungi and accurately select an antifungal agent.

Methods for diagnosis of tinea, including tinea unguium, include KOH direct microscopic examination in which the nail or cuticle is sampled from the affected area and observed under a microscope to confirm the presence or absence of fungal elements, and culturing methods in which the sample is cultured for several weeks on selective medium and fungal species are identified by observing the microstructure of colonies or cells. However, diagnosis by KOH direct microscopic examination requires skill, and basically does not allow identification of fungal species. Culturing methods, on the other hand, allow identification of fungal species but require long periods of time such as several weeks, have low culture-positive rates, and require skill for morphologic identification.

Another diagnosis method for tinea is, for example, one in which dermatophyte actin mRNA extracted from the nail plate of a tinea unguium patient is quantified by real-time PCR and it is attempted to detect the tinea fungus and estimate its volume (Non-patent document 2). However, this method lacks handling convenience because the target of detection is mRNA.

As yet another diagnosis method which allows accurate detection of causative fungi of fungus disease of the nail, there has been disclosed a method in which a first PCR and nested-PCR are conducted (Patent document 1). This method, however, requires electrophoresis and is not suitable for treatment of large amounts of specimen.

### Citation List

### Patent Literature

[Patent document 1] Japanese Unexamined Patent Application Publication No. 2008-067605

### Non-Patent Literature

[Non-patent document 1] Nishimoto, S.: Shinkinshi (Japanese Journal of Medical Mycology) 47:103-111, 2006
[Non-patent document 2] Tsuboi, R.: Rinpi (Japanese Journal of Clinical Dermatology) 57(5):94-97, 2003

Bergmans et al. (Clinical Microbiology And Infection 16(6):704-710, 2009) disclose a single-tube real-time PCR for detection and identification of 11 dermatophyte species in clinical material.

### Summary of Invention

### Technical Problem

Real-time PCR can be used for detection of viruses and pathogenic organisms. Real-time PCR is a method in which amplification of DNA by PCR and detection of amplification product by fluorescence are conducted simultaneously using a device integrating both a thermal cycler and a fluorescence detector. The method is advantageous because it does not require confirmation of amplification product by electrophoresis, allowing results to be obtained conveniently and rapidly, and also has low risk of contamination. However, no method has existed to date for sensitive and quantitative detection and identification of the causative fungi of tinea unguium by real-time PCR.

It is therefore an object of the present invention to provide a kit and method for identification of causative fungi of tinea unguium, which allows rapid and accurate identification of a causative fungus by real-time PCR using a primer set and a probe specific for a fungal species.

### Solution to Problem

As a result of diligent research directed toward solving the aforementioned problems, the present inventors have found that causative fungi of tinea unguium can be rapidly and accurately identified by real-time PCR using a primer set and probes specific for the ITS1 region in the ribosomal DNA of causative fungi of tinea unguium, and have completed this invention.

Specifically, the invention provides an identification kit for identification of causative fungi of tinea unguium using real-time PCR, which comprises a primer set and probes, wherein the primer set is at least one selected from the group consisting of a primer set consisting of a primer comprising the nucleotide sequence as set forth in SEQ ID NO: 1 and a primer comprising the nucleotide sequence as set forth in SEQ ID NO: 2, and a primer set consisting of a primer comprising the nucleotide sequence as set forth in SEQ ID NO: 3 and a primer comprising the nucleotide sequence as set forth in SEQ ID NO: 4, and wherein the probes comprise a probe comprising the nucleotide sequence as set forth in SEQ ID NO: 5 and a probe comprising the nucleotide sequence as set forth in SEQ ID NO: 6.

With a primer set consisting of a primer comprising the nucleotide sequence as set forth in SEQ ID NO: 1 and a primer comprising the nucleotide sequence as set forth in SEQ ID NO: 2, it is possible to amplify the full length of the ITS1 region of ribosomal DNA (approximately 350 bp) that is universal to fungi, and with a primer set consisting of a primer comprising the nucleotide sequence as set forth in SEQ ID NO: 3 and a primer comprising the nucleotide sequence as set forth in SEQ ID NO: 4 it is possible to specifically amplify a portion of the ITS1 region of ribosomal DNA (approximately 150 bp) of the major causative fungi, *Trichophyton rubrum* and *Trichophyton mentagrophytes.*

With a probe comprising the nucleotide sequence as set forth in SEQ ID NO: 5, it is possible to specifically detect the ITS1 region of ribosomal DNA of *Trichophyton mentagrophytes,* and with a probe comprising the nucleotide sequence as set forth in SEQ ID NO: 6 it is possible to specifically detect the ITS1 region of ribosomal DNA of *Trichophyton rubrum.*

Thus, with an identification kit of the invention it is possible to rapidly and accurately identify *Trichophyton rubrum* and *Trichophyton mentagrophytes,* which are the major causative fungi of tinea unguium.

The invention also provides a method for identifying causative fungi of tinea unguium, which comprises step of extracting total DNA from a nail sample taken from a subject, a step of preparing a primer set and probes specific to the ITS1 region of ribosomal DNA of a causative fungus of tinea unguium, and a step of amplifying the ITS 1 region of ribosomal DNA of the causative fungi of tinea unguium from the extracted total DNA using real-time PCR by the primer set, while simultaneously detecting the amplified DNA by the probes, and identifying the fungal species. According to the identification method of the invention, it is possible to rapidly and accurately identify causative fungi of tinea unguium.

In the identification method of the invention, the primer set is at least one selected from the group consisting of a primer set consisting of a primer comprising the nucleotide sequence as set forth in SEQ ID NO: 1 and a primer comprising the nucleotide sequence as set forth in SEQ ID NO: 2, and a primer set consisting of a primer comprising the nucleotide sequence as set forth in SEQ ID NO: 3 and a primer comprising the nucleotide sequence as set forth in SEQ ID NO: 4. With a primer set consisting of a primer comprising the nucleotide sequence as set forth in SEQ ID NO: 1 and a primer comprising the nucleotide sequence as set forth in SEQ ID NO: 2, it is possible to identify a wider range of causative fungi, and with a primer set consisting of a primer comprising the nucleotide sequence as set forth in SEQ ID NO: 3 and a primer comprising the nucleotide sequence as set forth in SEQ ID NO: 4, it is possible to accurately and efficiently identify the 2 major causative fungi of tinea unguium, namely *Trichophyton rubrum* and *Trichophyton mentagrophytes.* The primer set may also be used as a combination of 2 different types, depending on the purpose.

In the identification method of the invention, the probes comprise a probe comprising the nucleotide sequence as set forth in SEQ ID NO: 5 and a probe comprising the nucleotide sequence as set forth in SEQ ID NO: 6. The probes are a combination of 2 different types with which it is possible to simultaneously identify 2 different fungal species by a single real-time PCR procedure.

### Advantageous Effects of Invention

The identification kit and identification method of the invention do not require a high level of skill or a culturing time of several weeks, unlike the conventional KOH direct microscopic examination or culturing methods, and they therefore allow more convenient and rapid identification of causative fungi of tinea unguium. In addition, the quantitative efficiency, detection precision and fungal species specificity are improved even compared to the identification by conventional nested-PCR, and since no electrophoresis is necessary it is possible to significantly shorten the time required.

### Description of Embodiments

The identification method of the invention comprises a step of extracting total DNA from a nail sample taken from a subject, a step of preparing a primer set and a probe specific to the ITS1 region of ribosomal DNA of a causative fungus of tinea unguium, and a step of amplifying the ITS1 region of ribosomal DNA of the causative fungus of tinea unguium from the extracted total DNA using real-time PCR by the primer set, while simultaneously detecting the amplified DNA by the probes, and identifying the fungal species.

Causative fungi of tinea unguium include *Trichophyton rubrum, Trichophyton mentagrophytes, Trichophyton violaceum, Trichophyton tonsurans, Arthroderma vanbreuseghemii, Arthroderma benhamiae, Microsporum canis, Microsporum gypseum* and *Epidermophyton floccosum.* Since *Trichophyton rubrum* and *Trichophyton mentagrophytes* constitute over 90% of the isolated causative fungi of tinea unguium, the ability to identify these 2 fungal species is highly significant for treatment of tinea unguium.

The identification method of the invention uses as specimen a human nail from a patient having or suspected of having tinea unguium. The nail specimen may be taken by cutting using a nipper or clippers. The amount of nail specimen may be such as to allow extraction of a sufficient amount of DNA as template for real-time PCR (Real-time polymerase chain reaction). The thickness and humidity of the nail may vary, but an area of approximately 2 × 2 mm or an amount of about 5-10 mg may be taken.

Extraction of the total DNA from the nail specimen may be accomplished by a common method, such as pulverization of the nail with a bead shocker and sterilization by boiling at 100°C for 10 minutes, followed by extraction with phenol/chloroform and precipitation with ethanol. The extracted total DNA may be quantified if necessary using a commercially available nucleic acid quantitation kit.

The primer set to be used for the identification method of the invention is specific for the ITS1 (Internal Transcribed Spacer 1) region in ribosomal DNA of causative fungi of tinea unguium. The ITS1 region is a non-transcribed region, which has a more diversifiable DNA sequence in species evolution than other regions that are transcribed (such as the 28S region), and it is therefore believed to be a sequence that maintains high specificity for the fungal species. A person skilled in the art can appropriately design a primer set based on the nucleotide sequence of the ITS1 region in ribosomal DNA of causative fungi of tinea unguium, using the method described in Molecular Cloning: A Laboratory Manual (3rd Ed.) Cold Spring Harbor University Press, for example. The length of the primers is preferably 15-30 bp and more preferably 18-25 bp, and the GC content is preferably 40-60% and more preferably about 50%. The Tm value is preferably set to be 5°C to 10°C higher than the desired PCR annealing temperature, and since the annealing temperature is preferably 60°C to 65°C for the identification method of the invention, the Tm value is preferably 65°C to 70°C. The length of the DNA sequence to be amplified by the primer set may be 50-400 bp, and is preferably no greater than about 150 bp.

In order to identify a wide range of fungal species, it is preferred to use a primer set that is complementary to a specific sequence in the ITS1 region of fungal ribosomal DNA, and that specifically amplifies the ITS 1 region of ribosomal DNA that is universal to fungi. A primer set consisting of a primer comprising the nucleotide sequence as set forth in SEQ ID NO: 1 (dermaF) and a primer comprising the nucleotide sequence as set forth in SEQ ID NO: 2 (dermaR) allows amplification of the full length (approximately 350 bp) of the ITS1 region of ribosomal DNA of most fungi.

On the other hand, for efficient detection and identification targeted to *Trichophyton rubrum* and *Trichophyton mentagrophytes,* which are the major causative fungi that constitute over 90% of the causative fungi of tinea unguium, it is preferred to use a specific primer set limited to these fungal species. A primer set consisting of a primer comprising the nucleotide sequence as set forth in SEQ ID NO: 3 (dermaF2) and a primer comprising the nucleotide sequence as set forth in SEQ ID NO: 4 (dermaR2) allows specific amplification of a portion (approximately 150 bp) of the ITS 1 region of ribosomal DNA of *Trichophyton rubrum* and *Trichophyton mentagrophytes.* In addition, *Arthroderma vanbreuseghemii* and *Arthroderma simii,* which belong to same Group I in phylogenetic systematics as *Trichophyton mentagrophytes,* are also amplified by this primer set. Since the amplification product of this primer set has a shorter sequence than the amplification product of a universal primer set, the time required for real-time PCR can be shortened, and identification of the major causative fungi of tinea unguium can be accomplished accurately and efficiently.
SEQ ID NO: 1: 5'-TAACAAGGTTTCCGTAGGTGAACCT-3'
SEQ ID NO: 2: 5'-TCGCTGCGTTCTTCATCGA-3'
SEQ ID NO: 3: 5'-SSCCCCATTCTTGTCTACMTYAC-3'
SEQ ID NO: 4: 5'-AACGCTCAGACTGACAGCTCTTC-3'

The probes to be used for the identification method of the invention are specific for the ITS1 region in ribosomal DNA of causative fungi of tinea unguium. A person skilled in the art can find and design a probe specific to a fungal species based on the sequence of the ITS1 region in ribosomal DNA of different causative fungi of tinea unguium, using a known method such as the method of Wootton et al., for example (Federhen S., Analysis of compositionally biased regions in sequence databases. Methods Enzymol. 1996; 266:554-71). The length of the probe is not particularly restricted, and it may vary depending on the length of the nucleotide sequence of the specific region, and on the GC content and Tm value, but it is preferably a 10-50mer. The Tm of the probe is preferably higher than the Tm of the primer, and more preferably it is about 10°C higher. For a higher Tm and greater specificity, an MGB (Minor Groove Binder; product of Applied Biosystems, Japan) may be bound thereto.

A probe that specifically hybridizes to the ITS1 region in ribosomal DNA of *Trichophyton mentagrophytes* is the one having the sequence as set forth in SEQ ID NO: 5. With this probe it is possible to accurately identify whether or not a species is *Trichophyton mentagrophytes. Arthroderma vanbreuseghemii,* which is a sexual generation of *Trichophyton mentagrophytes,* can also be detected with this probe, and since both have the same clinical symptoms and method of treatment, there is no need for further discrimination and identification between them in the clinic. A probe that specifically hybridizes to the ITS1 region in ribosomal DNA of *Trichophyton rubrum* is the one having the sequence as set forth in SEQ ID NO: 6. With this probe it is possible to accurately identify whether or not a species is *Trichophyton rubrum. Trichophyton violaceum* also hybridizes with this probe. These two species can be discriminated by comparing the nucleotide sequences of the ITS1 regions (GeneBank Accession No. AB520840 and AB 194246) (for example, by sequencing of the PCR amplification products), but since *Trichophyton violaceum* is a very rare causative fungus of tinea unguium, it is essentially unnecessary to distinguish and identify them in the clinic. Also, a probe that specifically hybridizes to the ITS1 region in ribosomal DNA of all fungi is the one having the sequence as set forth in SEQ ID NO: 7. This probe allows detection of superficial fungi across a wide range, and quantitation allows confirmation of the total amount of superficial fungi or the proportion of causative fungi of tinea unguium of the total fungi.
SEQ ID NO: 5: 5'-CTCTCTTTAGTGGCTAAAC-3'
SEQ ID NO: 6: 5'-CGCGCTCCCCCTGC-3'
SEQ ID NO: 7: 5'-TTYAACAAYGGATCTCT-3'

The probes to be used in the identification method of the invention must be labeled for detection by real-time PCR. The labeled probe can be selected as desired by a person skilled in the art depending on the real-time PCR system, but TaqMan™ probe is preferably used. TaqMan™ probe is a probe labeled with a fluorescent dye at the 5'-end and with a quencher and MGB (Minor Groove Binder) at the 3'-end, of the nucleotide sequence that specifically hybridizes to the target DNA. The fluorescent dye may be FAM^{™}, VIC^{™}, NED^{™}, JOE^{™}, TAMRA^{™}, Cy-3, ROX^{™}, Texas Red^{™}, Cy-5 or the like.

The TaqMan™ probe, in its original state, has a quencher on the probe, and therefore emission of fluorescence is inhibited even when irradiated with excitation light. In real-time PCR amplification reaction, however, the TaqMan™ probe specifically hybridizes to template DNA in the annealing step, and when the TaqMan probe that has hybridized to the template is decomposed by the 5'→3' exonuclease activity of Taq DNA polymerase during the extension reaction step, the fluorescent dye is released from the probe and inhibition by the quencher is eliminated resulting in emission of fluorescence, which is detected with the real-time PCR system. Since the detected fluorescence intensity depends on the number of copies of target DNA in the sample, the fungus DNA in the specimen can thus be quantitated.

According to the invention, it is possible to select a primer set and probes suited for the purpose. For confirmation of major causative fungi, it is preferred to use a combination of a primer set consisting of a primer comprising the nucleotide sequence as set forth in SEQ ID NO: 3 and a primer comprising the nucleotide sequence as set forth in SEQ ID NO: 4, and probes having the sequence as set forth in SEQ ID NO: 5 and SEQ ID NO: 6. For identification of a wide range of fungi including rare causative fungi, it is preferred to use a combination of a primer set consisting of a primer comprising the nucleotide sequence as set forth in SEQ ID NO: 1 and a primer comprising the nucleotide sequence as set forth in SEQ ID NO: 2, and probes having the sequence as set forth in SEQ ID NO: 5 and SEQ ID NO: 6. Instead of a system using one primer set and one probe (single system), simultaneous identification of multiple fungal species can be accomplished using a system with multiple primer sets and multiple probes (multi system). With this type of multi system, the primers and probes can potentially interfere with each other and lower the detection sensitivity, and therefore the mixing ratios and concentrations of the primers and probes used must be investigated.

Identification of species of causative fungi can be carried out in the following manner. For a single system, when the fluorescent dye freed from the probe has been detected, the causative fungus being tested is judged to be a fungal species for which the probe is specific, and when the fluorescent dye has not been detected, the causative fungus being tested is judged not to be a fungal species for which the probe is specific. For a multi system, since the different probes are labeled with different fluorescent dyes, the type of fluorescent dye detected is used as the basis for determining that the causative fungus tested is a fungal species for which the probe labeled with that fluorescent dye is specific. An internal standard substance (internal control) may also be used to exclude false negatives due to failure of DNA extraction or purification. The internal control may be appropriately selected and modified by a person skilled in the art, and for example, an internal control for RNA, developed by Vollmer et al. (Evaluation of novel broad-range real-time PCR assay for rapid detection of human pathogenic fungi in various clinical specimens. J Clin Microbiol. 2008 Jun; 46(6): 1919-26. Epub 2008 Apr 2.) may be modified to one for DNA.

The identification kit of the invention is an identification kit comprising a primer set and probes for identification of causative fungi of tinea unguium using real-time PCR. The primer set is at least one selected from the group consisting of primer sets comprising a primer comprising the nucleotide sequence as set forth in SEQ ID NO: 1 and a primer comprising the nucleotide sequence as set forth in SEQ ID NO: 2, and primer sets comprising a primer comprising the nucleotide sequence as set forth in SEQ ID NO: 3 and a primer comprising the nucleotide sequence as set forth in SEQ ID NO: 4, and the probes comprise the probe comprising the nucleotide sequence as set forth in SEQ ID NO: 5 and the probe comprising the nucleotide sequence as set forth in SEQ ID NO: 6.

The identification kit of the invention may include, in addition to the aforementioned specific primer set and probe, also reagents generally included in real-time PCR kits, such as DNA polymerase, dNTP, buffer and positive control template. From the viewpoint of convenience of use, the reagents for PCR and fluorescence detection are preferably provided as a mixture with all in appropriate amounts.

The identification kit of the invention contains a combination of a primer set and probes suited for the object of identification. A kit used for identification of major causative fungi of tinea unguium is a combination of a primer set consisting of a primer comprising the nucleotide sequence as set forth in SEQ ID NO: 3 and a primer comprising the nucleotide sequence as set forth in SEQ ID NO: 4, and probes having the sequence as set forth in SEQ ID NO: 5 and SEQ ID NO: 6. A kit used for identification of rare causative fungi contains a primer set consisting of a primer comprising the nucleotide sequence as set forth in SEQ ID NO: 1 and a primer comprising the nucleotide sequence as set forth in SEQ ID NO: 2.

An identification kit containing multiple primer sets and probes is preferred as it allows identification of multiple causative fungi by a single real-time PCR procedure. In this case, in order to prevent reduction in detection sensitivity by interference between the primer sets or probes, the kit preferably contains the primer sets and probes in the optimal mixing ratios and concentrations determined by a positive control.

### Examples

The present invention will now be explained in greater detail with reference to examples, with the understanding that the invention is not meant to be limited to these examples.

(Example 1) Fungal species specificity of primer sets and probes Primer sets and probes specific for the sequence of ITS1 region in the ribosomal DNA of causative fungi of tinea unguium were used for real-time PCR, to examine fungal species specificity. Two different primer sets were used, (1) a primer set consisting of a primer comprising the nucleotide sequence as set forth in SEQ ID NO: 1 and a primer comprising the nucleotide sequence as set forth in SEQ ID NO: 2 (dermaF/dermaR), and (2) a primer set consisting of a primer comprising the nucleotide sequence as set forth in SEQ ID NO: 3 and a primer comprising the nucleotide sequence as set forth in SEQ ID NO: 4 (dermaF2/dermaR2). The primer set used for internal control was a primer set consisting of a primer comprising the nucleotide sequence as set forth in SEQ ID NO: 8 and a primer comprising the nucleotide sequence as set forth in SEQ ID NO: 9 (MS2-TM3-F/MS2-TM3-R). The primers were all purchased from Sigma Aldrich Japan, KK.
dermaF: 5'-TAACAAGGTTTCCGTAGGTGAACCT-3'
dermaR: 5'-TCGCTGCGTTCTTCATCGA-3'
dermaF2: 5'-SSCCCCATTCTTGTCTACMTYAC-3'
dermaR2: 5'-AACGCTCAGACTGACAGCTCTTC-3'
MS2-TM3-F: 5'- GGCTGCTCGCGGATACCC-3'
MS2-TM3-R: 5'-TGAGGGAATGTGGGAACCG -3'

Three different probes were used, (1) TaqMan™ probe labeled with FAM^{™} fluorescent dye at the 5'-end and with NFQ and MGB at the 3'-end of the nucleotide sequence as set forth in SEQ ID NO: 5 (TME-ITS1F), (2) TaqMan™ probe labeled with VIC^{™} fluorescent dye at the 5'-end and with NFQ and MGB at the 3'-end of the nucleotide sequence as set forth in SEQ ID NO: 6 (TRU-ITS1V) and (3) TaqMan™ probe labeled with NED^{™} fluorescent dye at the 5'-end and with NFQ and MGB at the 3'-end of the nucleotide sequence as set forth in SEQ ID NO: 7 (FU-ITS1N). (4) As the probe for internal control there was used TaqMan™ probe labeled with Cy5 fluorescent dye at the 5'-end and with BHQ3 at the 3'-end of the nucleotide sequence as set forth in SEQ ID NO: 10 (MS2-TM2-Cy5). NFQ (Non-fluorescent Quencher^{™}) and BHQ3 (Black Hole Quencher 3^{™}) are both non-fluorescent quenchers. The probes were purchased from Applied Biosystems, Japan and Sigma Aldrich Japan, KK.
TME-ITS1F: 5'-[FAM]-CTCTCTTTAGTGGCTAAAC-[NFQ-MGB]-3'
TRU-ITS1V: 5'-[VIC]CGCGCTCCCCCTGC-[NFQ-MGB]-3'
FU-ITS1N: 5'-[NED]TTYAACAAYGGATCTCT-[NFQ-MGB]-3'
MS2-TM2-Cy5:
   5'-[Cy5]-ACCTCGGGTTTCCGTCTTGCTCGT-[BHQ3]-3'

A variety of resident flora including dermatophytes were obtained from different microbial repositories. The fungal strains were cultured by common culturing methods and the total DNA was extracted by the phenol/chloroform method. The total DNA was quantitated with a Qubit quantitation kit by Invitrogen Corp., and 0.3 pg (an amount corresponding to approximately 100 copies of the ribosomal DNA gene) was used as template for real-time PCR using a 7500Fast Real-Time PCR system (Applied Biosystems, Japan). The composition of the reaction mixture (19 µL × 50 reaction portions) was as follows. A 19 µL portion was dispensed into each well, and the extracted DNA was added at 1 µL each.

| | |
|---|---|
| Water | 357 µL |
| Primers (30 µM) × 4 | 6.5 µL × 4 |
| ROX Reference Dye II | 20 µL |
| Probes (approx. 15 µM) × 4 | 20 µL × 4 |
| Premix | 500 µL |
| Total | 950 µL |

The procedure for the real-time PCR system was conducted according to the manufacturer's manual. The initial hold was 95°C for 30 seconds, and the PCR reaction was conducted for 60 cycles of 95°C for 10 seconds and 60°C for 30 seconds. The results of the real-time PCR are shown in Table 1. Results with amplification (positive) are indicated by "+", and results without amplification (negative) are indicated as "-" .

**[Table 1]**

| Primer pairs | DermaF/dermaR | | | DermaF2/dermaR 2 | |
|---|---|---|---|---|---|
| Probes | FU-IT S1N | TRU-ITS1V | TME-ITS1F | TRU-ITS1V | TME-ITS1F |
| Dermatophytes | | | | | |
| *Trichophyton rubrum* T99 | + | + | - | + | - |
| *Trichophyton rubrum* T108 | + | + | - | + | - |
| *Trichophyton rubrum* 4-21 (Hasegawa) | + | + | - | + | - |
| *Trichophyton rubrum* 4-23 (Hasegawa) | + | + | - | + | - |
| *Trichophyton rubrum* 4-25 (Hasegawa) | + | + | - | + | - |
| *Trichophyton rubrum* 4-26 (Haserawa) | + | + | - | + | - |
| *Trichophyton mentagrophytes* T7 | + | - | + | - | + |
| *Trichophyton mentagrophytes* T9 | + | - | + | - | + |
| *Arthroderma vanbreuseghemii* TIMM2789 | + | - | + | - | + |
| *Trichophyton tonrurans* T117 | + | - | - | - | - |
| *Arthroderma benhamiae* SM103 | + | - | - | - | - |
| *Microsporum canis* TDGS945 | + | - | - | - | - |
| *Microsporum canis* TDGS0222 | + | - | - | - | - |
| *Microsporum canis* TDGS0223 | + | - | - | - | - |
| *Microsporum canis* TDGS0269 | + | - | - | - | - |
| *Microsporum gypsum* TDGS0009 | + | - | - | - | - |
| *Microsoorum gypsum* TDGS0012 | + | - | - | - | - |
| *Epidermophyton floccosum* 5-14 (Hasegawa) | + | - | - | - | - |
| *Epidermophyton floccosum* 5-22 (Hasegawa) | + | - | - | - | - |
| Other pathogenic fungi | | | | | |
| *Candida albicans* ATCC10231 | + | - | - | - | - |
| *Candida albicans* TIMM1768 | + | - | - | - | - |
| *Candida albicans* ATCC90028 | + | - | - | - | - |
| *Candida glabrata* ATCC90030 | + | - | - | - | - |
| *Candida glabrata* CBS138 | + | - | - | - | - |
| *Candida tropicalis* ATCC750 | + | - | - | - | - |
| *Candida tropicalis* TLCS S 9/1 | + | - | - | - | - |
| *Candid parapsilosis* ATCC2209 | + | - | - | - | - |
| *Candida parapsilosis* ATCC90018 | + | - | - | - | - |
| *Aspergillus fumigatus* TIMM0108 | + | - | - | - | - |
| *Aspergillus fumigatus* JCM10253 | + | - | - | - | - |
| *Aspergillus fumigatus* TIMM2920 | + | - | - | - | - |
| *Aspergillus flavus* TIMM0059 | + | - | - | - | - |
| *Aspergillus flavus* TIMM2935 | + | - | - | - | - |
| *Aspergillus niger* TIMM0113 | + | - | - | - | - |
| *Aspergillus niger* TIMM2915 | + | - | - | - | - |
| *Paecilomyces variotii* TIMM3182 | + | - | - | - | - |
| *Pseudallescherichia boydii* TIMM0886 | + | - | - | - | - |
| *Fusarium oxysporum* TSY-0351 | + | - | - | - | - |
| *Fusarium solani* TSY-0403 | + | - | - | - | - |
| *Fusarium verticillioides* TSY-0219 | + | - | - | - | - |
| *Chaetomium globosum* TSY-0369 | + | - | - | - | - |
| *Exophiala jeanselmei* TSY-0396 | + | - | - | - | - |
| *Cryptococcus neoformans* ATCC90113 | + | - | - | - | - |
| *Crynlococcus neoformans* TJane | + | - | - | - | - |
| *Trichosporon asahii* CBS2479 | + | - | - | - | - |
| *Rhizopus oryzae* TIMM0921 | + | - | - | - | - |

Based on Table 1, a wide range of fungi were detected in this detection system with the dermaF/dermaR primers and FU-ITS1N probe, which amplify the full length of the ITS1 region. Also, with the dermaF/dermaR or dermaF2/dermaR2 and probe set, both *Trichophyton mentagrophytes* and *Trichophyton rubrum* were specifically detected. The fungal species-specific probes reacted only with the specific fungal species, and did not react at all with other fungal species (100% species-specific).

### (Example 2) Detection sensitivity in real-time PCR

A primer set and a probe specific for the sequence of the ITS1 region in the ribosomal DNA of causative fungi of tinea unguium were used to examine the detection sensitivity of the identification method of the invention. As positive controls there were used the ITS1 regions of *Trichophyton rubrum, Trichophyton mentagrophytes* and *Aspergillus fumigatus,* cloned in the pCR2.1 vector included in the TOPO TA Cloning Kit by Invitrogen Corp. The template concentration was diluted to determine the lower limit of detection sensitivity.

In the detection system, the reproducibility was confirmed by 4 repeated replications for each concentration. The results are shown in Table 2. A single amplification is indicated by "+", and amplifications that occurred for all of the 4 measurements are indicated by "++++".

**[Table 2]**

| Primer pairs | DermaF/dermaR | | | DermaF2/dermaR2 | |
|---|---|---|---|---|---|
| Probes | FU-ITS1N | TRU-ITS1V | TME-ITS1F | TRU-ITS1V | TME-ITS1F |
| Number of rDNA copies | | | | | |
| 400 | ++++ | ++++ | ++++ | ++++ | ++++ |
| 200 | ++++ | ++++ | ++++ | ++++ | ++++ |
| 100 | ++++ | ++++ | ++++ | ++++ | ++++ |
| 50 | ++++ | ++++ | ++++ | ++++ | ++++ |
| 25 | ++++ | + | ++++ | ++++ | ++++ |
| 13 | ++++ | ++ | ++++ | +++ | ++++ |
| 6 | +++ | + | +++ | + | ++++ |
| 3 | +++ | - | +++ | - | +++ |

In regions with very dilute template, a probabilistic PCR amplification model (Poisson process) involving a limited time and small number of molecules is applied, and this is shown by a probabilistic value, or detectability factor, instead of negative/positive. While the value will vary depending on the detection system and situation, 90-95% is applied as the borderline for detection sensitivity in a common real-time PCR detection system, and therefore in this detection system, the template concentration detected at 100% was taken as the minimum detection sensitivity. In this detection system, 3 to 50 copies of ribosomal DNA template produced results of 100% amplification. Since this corresponds to 1-2 cells of fungi such as Trichophyton species, it is presumed to be adequate sensitivity for practical use. Also, this detection sensitivity was found to be 2- to 166-fold higher sensitivity than with nested-PCR, which requires 100-500 copies.

### (Example 3 Clinical trial using identification method of the invention)

Dermatology patients with untreated onychomycosis were directly observed by a skilled doctor using a microscope, and the presence or absence of infection was confirmed. The nails of patients confirmed to have infection were disinfected with ethanol, and then the distal end of each nail was sampled by cutting with clippers or a nipper. The sampled nails were crushed with a Multi-beads shocker (cell disruptor) (Yasui Kikai Corp.) and boiled for 10 minutes at 100°C in filamentous fungi buffer (200 mM Tris-HCl, pH 8.0, 25 mM EDTA, 0.5% SDS, 250 mM NaCl), and then subjected to phenol/chloroform extraction followed by ethanol precipitation for extraction of the DNA. A 50 µL portion of the DNA was dissolved in ultrapure water and stored. A 25 µL portion of the obtained DNA-containing solution was used as a template DNA sample for real-time PCR in the same manner as Example 1, together with a positive control of known concentration.

The results are shown in Table 3. In the table, C_{T} is data obtained from the amplification curve, and it represents the number of PCR cycles corresponding to the set threshold value. The amount of template can be quantitatively estimated from C_{T} by either the calibration curve method or comparative C_{T} method. Here, the comparative C_{T} method was used, which is suitable for large amounts of specimen, and the number of templates were calculated from it. The comparative C_{T} method assumes 100% PCR efficiency, and the number of templates is estimated utilizing the difference in C_{T} from a known positive control. This method has the disadvantage of quantitative error due to differences in amplification efficiency, but the problem is considered negligible for PCR which exhibits logarithmic behavior. Upon confirming the amplification efficiency for the clinical specimens with LinRegPCR software (Ruijter JM et al., Amplification efficiency: linking baseline and bias in the analysis of quantitative PCR data. Nucleic Acids Res. 2009 Apr; 37(6):e45. Epub 2009 Feb 22), virtually no difference was found, and it was therefore judged that the amplification efficiency using the comparative C_{T} method was not of a problematic level and permitted the number of template copies to be estimated.

The identification method of the invention was applied for nails from 7 healthy individuals, and since only a maximum of about 100 copies of fungal template was detected, it was assumed that about 100 copies are normally resident, and therefore values above this were used for diagnosis of tinea unguium. This level was also believed to be appropriate because the amounts of template detected in actual clinical specimens were all well above 100 copies.

According to Table 3, all of the tinea unguium specimens in 33 cases judged to be positive by direct microscopic observation by a skilled doctor were diagnosed as positive by the identification method of the invention (detection accuracy: 100%). On the other hand, 15 specimens were identified as fungal species by culturing, of which 13 matched the determination results of the identification method of the invention. It is believed that the 2 non-matching cases may have been misidentified in culturing, since the identification method of the invention has 100% species specificity. Thus, fungal species identification by culturing was 39% while identification accuracy according to the invention was 100%. In addition, the remaining 16 cases that could not be identified as fungal species by culturing were all identified as fungal species. However, when the identification method of the invention was applied to healthy nails, absolutely no signal was detected for Trichophyton species (all 7 cases), indicating an absence of false-positive results. In Table 3, TR represents *Trichophyton rubrum,* TM represents *Trichophyton mentagrophytes,* ND represents "Not detected", and ITS1 indicates that no identification was made despite appearance of an amplification band.

**[Table 3]**

| Specimen (No.) | Physician observations | | | Judgment in RT-PCR | DermaF/dermaR | | | DermaF2/dermaR2 | |
|---|---|---|---|---|---|---|---|---|---|
| | Microscopic examination | Culturing | Ordinary PCR | | FU-ITS1N | TME-ITS1F | TRU-ITS1V | TME-ITS1F | TRU-ITS1V |
| 47 | + | - | ND | TR | 64 | - | 195 | - | 6250 |
| 49 | + | - | ND | TR | 25000 | - | 200000 | - | 400000 |
| 54 | + | Unidenti-fiabl e | TR | TR | 391 | - | 3125 | - | 6250 |
| 60 | + | TR | TR | TR | 50000 | - | 100000 | - | 400000 |
| 64 | + | TR | TM | TM | 3125 | 1563 | - | 50000 | - |
| 66 | + | - | ITS1 | TR | 12500 | - | 25000 | - | 391 |
| 68 | + | CA | ND | TR | 12500 | - | 50000 | - | 200000 |
| 71 | + | TM | TM | TM | 3125 | 125000 | - | 100000 | - |
| 86 | + | - | ND | TR | 195 | - | 781 | - | 125000 |
| 95 | + | - | ND | TR | 391 | | 781 | - | 125000 |
| 110 | + | Poor growth | TM | TM | 49 | 98 | - | 3125 | - |
| 114 | + | TR | TM | TM | 3125 | 6250 | - | 100000 | - |
| 118 | + | - | ND | TR | 49 | 12 | 48 | 98 | 391 |
| | | | | TM | | | | | |
| 121 | + | TR | TR | TR | 24 | - | 98 | - | 781 |
| | | | TM | | | | | | |
| 130 | + | - | ND | TR | 98 | - | 781 | - | 125000 |
| 140 | + | - | ND | TR | 125000 | - | 12500 | - | 50000 |
| 142 | + | TR | TR | TR | 49 | - | - | - | 49 |
| | | | TM | | | | | | |
| 146 | + | - | ND | TR | 781 | - | 781 | - | 6250 |
| 154 | + | - | ND | TR | 20000 | - | 80000 | - | 20000 |
| 164 | + | - | ND | TR | 3125 | - | 6250 | - | 6250 |
| 167 | + | TR | TR | TR | 24 | - | 24 | - | 98 |
| 170 | + | - | ND | TR | 781 | - | 391 | - | 3125 |
| 172 | + | TR | TR | TR | 3125 | - | 3125 | - | 50000 |
| 175 | + | TR | TR | TR | 1000000 | - | 100000 | - | 100000 |
| 178 | + | TR | TR | TR | 49 | - | 12 | - | 781 |
| | | | TM | | | | | | |
| 182 | + | TR | TR | TR | 25000 | - | 25000 | - | 100000 |
| | | | TM | | | | | | |
| 185 | + | - | ND | TR | 1563 | - | 1563 | 98 | 3125 |
| | | | | TM | | | | | |
| 191 | + | - | ND | TR | 98 | - | 98 | - | 391 |
| 195 | + | TR | TR | TR | 1563 | - | 3125 | - | 100000 |
| 206 | + | - | ND | TR | 3 | - | - | - | 391 |
| 210 | + | TR | TR | TR | 3125 | - | 3125 | - | 50000 |
| 230 | + | - | ND | TR | 50000 | - | 25000 | - | 25000 |
| 237 | + | TR | TR | TR | 25000 | - | 25000 | - | 25000 |
| Negative | | | | | - | - | - | - | - |
| Positive | | | | | 100000 | 100000 | 100000 | 100000 | 100000 |

The results of identifying causative fungi of the tinea pedis specimens by the same method are shown in Table 4.

**[Table 4]**

| Specimen (No.) | Physician observations | | | Judgment in RT-PCR | DermaF/dermaR | | | DermaF2/dermaR2 | |
|---|---|---|---|---|---|---|---|---|---|
| | Microscopic examination | Culturing | Ordinary PCR | | FU-ITS1N | TME-ITS1F | TRU-ITS1V | TME-ITS1F | TRU-ITS1V |
| 49 nail | + | TR | TR | TR | 50000 | - | 100000 | - | 400000 |
| 49 skin | + | - | ND | TR | 49 | - | 195 | - | 781 |
| 56 nail | + | TM | TM | TM | 3125 | 125000 | - | 100000 | - |
| 56 skin | + | TM | TM | TM | 25000 | 25000 | - | 100000 | - |
| 69 nail | + | - | ND | TR | 195 | - | 781 | - | 12500 |
| 69 skin | + | - | ITS1 | TR | 3125 | - | 50000 | - | 200000 |
| 88 nail | + | Poor growth | TM | TM | 49 | 98 | - | 3125 | - |
| 88 skin | + | TM | TM | TM | 195 | 195 | - | 1563 | - |
| 91 nail | + | TR | TM | TM | 3125 | 6250 | - | 100000 | - |
| 91 skin | + | TM | TM | TM | 6250 | 6250 | - | 25000 | - |
| 95 nail | + | - | ND | TR | 391 | - | 781 | - | 125000 |
| 95 skin | + | TM | TM | TM | 1563 | 1563 | - | 25000 | - |
| 121 nail | + | TR | TR | TR | 24 | - | 98 | - | 781 |
| | | | TM | | | | | | |
| 121 skin | + | - | ND | TR | 781 | - | 781 | - | 6250 |
| 127 nail | + | - | ND | TR | 20000 | - | 80000 | - | 20000 |
| 127 skin | + | - | ND | TR | 12500 | 12500 | 1563 | 100000 | 100000 |
| | | | | TM | | | | | |
| 143 nail | + | - | ND | TR | 781 | - | 391 | - | 3125 |
| 143 skin | + | TR | TR | Fungus | 24 | - | - | - | 781 |
| 145 nail | + | TR | TR | TR | 3125 | - | 3125 | - | 50000 |
| 145 skin | + | TR | TR | TR | 3125 | - | 50000 | - | 400000 |
| Negative | | | | | - | - | - | - | - |
| Positive | | | | | 100000 | 100000 | 100000 | 100000 | 100000 |

### Industrial Applicability

According to the identification method of the invention, it is possible to rapidly and accurately identify causative fungi of tinea unguium, and to provide effective data on causative fungi for development of courses of treatment, and therefore the industrial applicability is significant.

## Claims

1. An identification kit for identification of causative fungi of tinea unguium using real-time PCR, which comprises at least one primer set and probes,
wherein the primer set is at least one selected from the group consisting of a primer set consisting of a primer comprising the nucleotide sequence as set forth in SEQ ID NO: 1 and a primer comprising the nucleotide sequence as set forth in SEQ ID NO: 2, and a primer set consisting of a primer comprising the nucleotide sequence as set forth in SEQ ID NO: 3 and a primer comprising the nucleotide sequence as set forth in SEQ ID NO: 4, and
wherein the probes comprise a probe comprising the nucleotide sequence as set forth in SEQ ID NO: 5 and a probe comprising the nucleotide sequence as set forth in SEQ ID NO: 6.

2. A method for identifying causative fungi of tinea unguium, which comprises:
a step of extracting total DNA from a nail sample taken from a subject,
a step of preparing a primer set and probes specific to the ITS1 region of ribosomal DNA of a causative fungus of tinea unguium, and
a step of amplifying the ITS1 region of ribosomal DNA of the causative fungus of tinea unguium from the extracted total DNA using real-time PCR by the primer set, while simultaneously detecting the amplified DNA by the probes, and identifying the fungal species;
wherein the primer set is at least one selected from the group consisting of a primer set consisting of a primer comprising the nucleotide sequence as set forth in SEQ ID NO: 1 and a primer comprising the nucleotide sequence as set forth in SEQ ID NO: 2, and a primer set consisting of a primer comprising the nucleotide sequence as set forth in SEQ ID NO: 3 and a primer comprising the nucleotide sequence as set forth in SEQ ID NO: 4, and
wherein the probes comprise a probe comprising the nucleotide sequence as set forth in SEQ ID NO: 5 that specifically hybridizes to the ITS1 region in the ribosomal DNA of *Trichophyton mentagrophytes,* and a probe comprising the nucleotide sequence as set forth in SEQ ID NO: 6 that specifically hybridizes to the ITS1 region in the ribosomal DNA of *Trichophyton rubrum.*

## Patentansprüche

1. Identifizierungskit zur Identifizierung von Pilzen, die Tinea unguium verursachen, unter Verwendung von Real-Time-PCR, der mindestens einen Primersatz und Sonden umfasst,
wobei es sich bei dem Primersatz um mindestens einen handelt, ausgewählt aus der Gruppe, bestehend aus einem Primersatz, der aus einem Primer, umfassend die in SEQ ID NO: 1 dargelegte Nucleotidsequenz, und einem Primer, umfassend die in SEQ ID NO: 2 dargelegte Nucleotidsequenz, besteht, und einem Primersatz, der aus einem Primer, umfassend die in SEQ ID NO: 3 dargelegte Nucleotidsequenz, und einem Primer, umfassend die in SEQ ID NO: 4 dargelegte Nucleotidsequenz, besteht, und
wobei die Sonden eine Sonde, umfassend die in SEQ ID NO: 5 dargelegte Nucleotidsequenz, und eine Sonde, umfassend die in SEQ ID NO: 6 dargelegte Nucleotidsequenz, umfassen.

2. Verfahren zum Identifizieren von Pilzen, die Tinea unguium verursachen, welches umfasst:
einen Schritt, bestehend im Extrahieren von Gesamt-DNA aus einer von einem Probanden entnommenen Nagelprobe,
einen Schritt, bestehend im Herstellen eines Primersatzes und von Sonden, die spezifisch für die ITS1-Region ribosomaler DNA eines Pilzes sind, der Tinea unguium verursacht, und
einen Schritt, bestehend im Amplifizieren der ITS1-Region ribosomaler DNA des Pilzes, der Tinea unguium verursacht, aus der extrahierten Gesamt-DNA unter Verwendung von Real-Time-PCR mit dem Primersatz, während gleichzeitig die amplifizierte DNA durch die Sonden nachgewiesen wird, und Identifizieren der Pilzart;
wobei es sich bei dem Primersatz um mindestens einen handelt, ausgewählt aus der Gruppe, bestehend aus einem Primersatz, der aus einem Primer, umfassend die in SEQ ID NO: 1 dargelegte Nucleotidsequenz, und einem Primer, umfassend die in SEQ ID NO: 2 dargelegte Nucleotidsequenz, besteht, und einem Primersatz, der aus einem Primer, umfassend die in SEQ ID NO: 3 dargelegte Nucleotidsequenz, und einem Primer, umfassend die in SEQ ID NO: 4 dargelegte Nucleotidsequenz, besteht, und
wobei die Sonden eine Sonde, umfassend die in SEQ ID NO: 5 dargelegte Nucleotidsequenz, die spezifisch mit der ITS1-Region in der ribosomalen DNA von *Trichophyton mentagrophytes* hybridisiert, und eine Sonde, umfassend die in SEQ ID NO: 6 dargelegte Nucleotidsequenz, die spezifisch mit der ITS1-Region in der ribosomalen DNA von *Trichophyton rubrum* hybridisiert, umfassen.

## Revendications

1. Trousse d'identification pour l'identification des champignons agents causaux de Tinea unguium en utilisant la PCR en temps réel, qui comprend au moins un jeu d'amorces et des sondes,
dans laquelle le jeu d'amorces est au moins l'un sélectionné parmi le groupe constitué d'un jeu d'amorces constitué d'une amorce comprenant la séquence nucléotidique telle qu'exposée dans SEQ ID n° : 1 et d'une amorce comprenant la séquence nucléotidique telle qu'exposée dans SEQ ID n° : 2, et un jeu d'amorces constitué d'une amorce comprenant la séquence nucléotidique telle qu'exposée dans SEQ ID n° : 3 et d'une amorce comprenant la séquence nucléotidique telle qu'exposée dans SEQ ID n° : 4, et
dans laquelle les sondes comprennent une sonde comprenant la séquence nucléotidique telle qu'exposée dans SEQ ID n° : 5 et une sonde comprenant la séquence nucléotidique telle qu'exposée dans SEQ ID n° : 6.

2. Procédé d'identification des champignons agents causaux de Tinea unguium, qui comprend :
une étape d'extraction de l'ADN total d'un prélèvement d'ongle effectué chez un sujet,
une étape de préparation d'un jeu d'amorces et des sondes spécifiques de la région ITS1 de l'ADN ribosomique d'un champignon agent causal de Tinea unguium, et
une étape d'amplification de la région ITS1 de l'ADN ribosomique du champignon agent causal de Tinea unguium à partir de l'ADN total extrait en utilisant la PCR en temps réel par le jeu d'amorces, tout en détectant simultanément l'ADN amplifié par les sondes, et en identifiant les espèces fongiques ;
dans laquelle le jeu d'amorces est au moins l'un sélectionné parmi le groupe constitué d'un jeu d'amorces constitué d'une amorce comprenant la séquence nucléotidique telle qu'exposée dans SEQ ID n° : 1 et d'une amorce comprenant la séquence nucléotidique telle qu'exposée dans SEQ ID n° : 2, et d'un jeu d'amorces constitué d'une amorce comprenant la séquence nucléotidique telle qu'exposée dans SEQ ID n° : 3 et d'une amorce comprenant la séquence nucléotidique telle qu'exposée dans SEQ ID n° : 4, et
dans laquelle les sondes comprennent une sonde comprenant la séquence nucléotidique telle qu'exposée dans SEQ ID n° : 5 qui s'hybride spécifiquement à la région ITS1 de l'ADN ribosomique de *Trichophyton mentagrophytes,* et une sonde comprenant la séquence nucléotidique telle qu'exposée dans SEQ ID n° : 6 qui s'hybride spécifiquement à la région ITS1 de l'ADN ribosomique de *Trichophyton rubrum.*
